# EUROPEAN PATENT APPLICATION

(11) **EP 2 289 882 A1**
(43) Date of publication of application: **02.03.2011**
(21) Application number: 09169036.2
(22) Date of filing: 31.08.2009
(51) Int. Cl.: C07D 241/08, C07D 403/12, C07D 471/10, A61K 31/496, A61K 31/499, A61K 31/495, A61P 25/28

(54) **3-oxopiperazinium derivatives as agonists of nerve growth factor and their use as medicaments**

(71) Applicant: Institut d'Investigacions Biomédiques August PI I Sunyer, 08036 Barcelona (ES); CONSEJO SUPERIOR DE INVESTIGACIONES CIENTIFICAS, 28006 Madrid (ES)
(72) Inventor: Moreno, Beatriz, 08036, Barcelona (ES); Villoslada, Pablo, 08036, Barcelona (ES); Messeguer, Joaquín, 28006, Madrid (ES); Navarro, Gloria, 28006, Madrid (ES); Messeguer, Angel, 28006, Madrid (ES)
(74) Representative: Illescas, Manuel

(57) **Abstract**

New 3-oxopiperazinium derivatives agonists of Nerve Growth Factor and it use as medicaments. Neurotrophin binding to its specific receptor Trk A leads to the activation of multiple signalling cascades, culminating in neuroregenerative effects, including neuronal survival and neurite outgrowth. Neurotrophic factors have been used for the treatment of several neurodegenerative diseases. However, their use is limited by their inability to cross the blood-brain barrier, their short half life and their side effects. Small molecule neurotrophin peptidomimetics may be beneficial in treating a number of neurodegenerative disorders. The present invention shows the capacity of nerve growth factor agonist molecules derived from the 3-oxopiperazinium scaffold to induce differentiation in PC12 cells and, therefore, that these small molecules with NGF agonist activity may be beneficial for treatment of neurodegenerative diseases, due to their neuroregenerative effects.

## Description

### Field of invention

This invention applies to the area of therapeutics for neurological, psychiatric disorders, and ageing. In particular, it relates to the neuroprotective effect of small molecule agonists of neurotrophin (Nervous Growth Factor -NGF-) and the use of those agonists as medicaments.

### Background of the invention

Ageing, neurological and psychiatric disorders cause death and damage to nerve cells. Frequent and relevant insults to the nervous system include neuronal degeneration, ischemia, inflammation, immune responses, trauma, and cancer, among other. As a consequence of these, nerve cells can die within minutes or hours or survive to this initial insult in an impaired state that activates neurodegeneration, ending equally in cellular death.

Given the importance of the nervous system in enabling basic motor skills and sensing, there exists an interest in finding therapeutic weapons to protect the nervous system.

Neuroprotection aims to the preservation, recovery, cure, or regeneration of the nervous system, its cells, structure, and function (Vajda et al., 2002). A goal of neuroprotection is to prevent or minimize the effects of an original insult to the nervous system, or to prevent or minimize the consequences of endogenous or exogenous noxious processes causing damage to axons, neurons, synapses, and dendrites.

Treatment strategies in general are frequently based on the modulation of a single proposed injury factor. Although such treatments can be shown to be beneficial in highly constrained animal models, they are less likely to prove efficacious in the more complex human disorder that involves more variable degrees of injury severity in a genetically diverse population (Faden and Stoica, 2007). Importantly, since the presumed mechanisms of neuronal death are both complex and varied, such as oxidative stress, mitochondrial dysfunction, protein aggregation, apoptosis, and inflammation (Youdim et al., 2005), single compounds having multipotential effects on multiple injury mechanisms are desirable.

Several neuroprotective drugs are under investigation including the following classes: anti-inflammatory agents, N-methyl D-aspartate (NMDA) antagonists, α-amino-3-hydroxy-5-methyl-4-isoxazole propionic acid (AMPA) antagonists, dexanabinol, sodium channel blockers, thyrotropin-releasing hormone (TRH), growth factors, glucocorticoids, caffeinol, opioid antagonists, apoptosis inhibitors, free radical trappers/scavengers, erythropoietin, calcium channel blockers, magnesium sulfate, statins.

The ability of these pharmacological agents to limit secondary biochemical damage and cell death has been well established in numerous animal models of stroke, head injury, and spinal cord injury, yet the results of such neuroprotective treatment strategies in human injury have been disappointing (Faden and Stoica, 2007).

Neurotrophins are growth factors that regulate the development and maintenance of the peripheral and the central nervous systems (Lewin and Barde, 1996). Nerve growth factor (NGF) is a homodimeric protein from the neurotrophin family that plays a crucial role in neuronal survival, differentiation and growth (Levi-Montalcini, 1987) and binds two distinct cellular receptors: the tyrosine kinase receptor TrkA and the p75 receptor (Chao, 2003). NGF-TrkA binding activates the intrinsic tyrosine kinase of the receptor, causing tyrosine phosphorylation of TrkA and associated signalling partners and therefore activating promotion of cell survival or differentiation (Kaplan and Miller, 2000). The p75 receptor is a member of the tumor necrosis factor receptor superfamily. Depending on the cellular environment and the type of ligand, p75 can act as transducer of pro-survival, pro-apoptotic, or pro-differentiation signals (Barker, 1998; Rabizadeh et al., 1999; Zaccaro et al., 2001; Saragovi and Zaccaro, 2002). Accordingly, depending of the metabolic route, binding to either TrkA or p75 receptors may trigger signals, depending on the cell type considered, linked to, indistinctly, differentiation and/or cell survival.

The potential of NGF as a therapeutic agent for several diseases has been indicated by several investigators. Such diseases include neurodegenerative disorders, nerve inflammation and certain types of cancers, multiple sclerosis, neuromyelitis optica, amyotrophic lateral sclerosis (ALS), Parkinson's disease, Alzheimer's disease, Friedreich's ataxia, Huntington's disease, Dementia with Lewy bodies, spinal muscular atrophy, major depressive disorder, schizophrenia, glaucoma or peripheral neuropathies (diabetic or AIDS neuropathy) (Longo et al, 2007; Schulte-Herbrüggen, 2007; Shi, 2007; Hellveg, 2008; Shoval, 2005; Apfel, 2002; Anand, 2004). NGF has significant immunoregulatory properties during CNS inflammation to contribute to the maintenance of the CNS privilege (Villoslada and Genain, 2004). During EAE in marmoset, NGF was able to inhibit the development of the clinical symptoms when administered intracerebroventricularly by continuous infusion apparently because of its ability to induce an immunosuppressive microenvironment in the CNS which leads to decreased CNS infiltration (Villoslada et al., 2000). The finding that NGF induces immunosuppression during autoimmune demyelination in addition to its neuroprotective properties in neurons and oligodendrocytes makes it a very good candidate for the treatment of CNS inflammatory diseases like MS. However, NGF is not the ideal drug candidate due to its inability to cross the blood-brain barrier (BBB) (Poduslo and Curran, 1996), its short half life and its side effects (Apfel, 2002). Much effort has been made in the search for small molecules with NGF agonist activity, with better pharmacokinetics and less side effects. To achieve this goal, different approaches have been attempted (Poduslo and Curran, 1996; Longo et al., 1997; Maliartchouk et al., 2000a; Maliartchouk et al., 2000b; Peleshok and Saragovi, 2006).

As such, there is an ongoing need for providing drugs, particularly NGF agonists, with neuroprotective properties, which have preferably multipotential effects, but without the drawbacks of NGF. Present invention has developed a family of compounds alternative to those disclosed in the state of the art. The family of compounds of the invention are all peptidomimetics of NGF, agonists to Trk A specific receptor binding.

They are all 3-oxopiperazinium derivatives which share a cation charge due to a tetrasubstituted nitrogen atom present in its structure.

### Summary of the invention

The present invention relates to compounds of Formula I and their pharmaceutically acceptable salts and/or prodrugs. 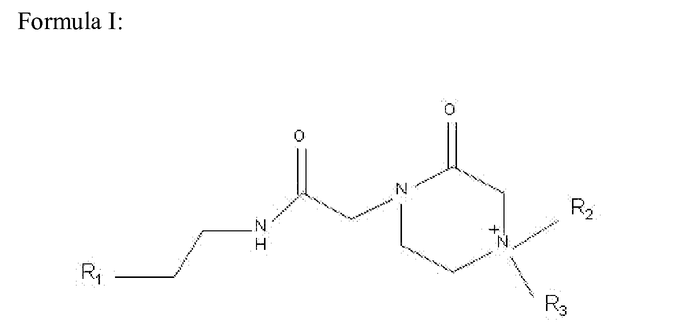 wherein 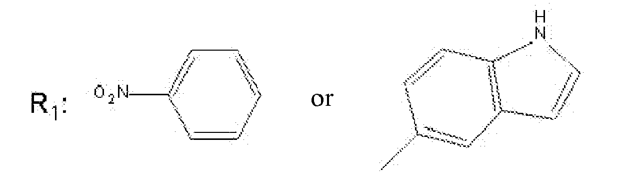 R₂ and R₃ are each one independently 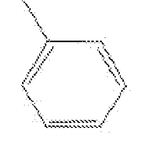 or CH₃
or form together with the 3-oxopiperazinium ring a fused azoniosapiro [4,5] decane heterocycle.

The invention also covers the use of the compounds of Formula I, its pharmaceutically acceptable salts and/or prodrugs thereof, as active ingredients in the manufacture of medicaments for the prevention or treatment of nerve cell death or damage. In other words, the present invention relates to the compounds of Formula I, its pharmaceutically acceptable salts and/or prodrugs thereof for use in the prevention or treatment of nerve cell death or damage. Similarly, the present invention relates to a method of neuroprotection comprising administering to a subject in need thereof an effective amount of a compound of Formula I, its pharmaceutically acceptable salts and/or prodrugs thereof.

The term "prodrug", as used herein, includes any compound derived from the compounds of Formula I, for example, the ester, amide, phosphate, etc., which, upon being administered to an individual, is capable of providing the compounds of Formula I or the pharmaceutically acceptable salt thereof, directly or indirectly, to said individual. Preferably, said derivative is a compound that increases the bioavailability of the compounds of Formula I when administered to an individual or that promotes the release of the compounds of Formula I in a biological compartment. The nature of said derivative is not critical, provided that it may be administered to an individual and that it provides the compounds of Formula I in an individual's biological compartment. The preparation of said prodrug may be performed by conventional methods known by those skilled in the art. An example of prodrug of the compounds of Formula I can be their encapsulation into liposomes.

The term "pharmaceutically acceptable" means that a compound or combination of compounds is sufficiently compatible with the other ingredients of a formulation, and not deleterious to the patient up to those levels acceptable by the industry standards.

For therapeutic use, salts of the compounds of Formula I are those wherein the counter-ion is pharmaceutically acceptable.

The term salt as mentioned herein is meant to comprise any stable salts, which the compounds of Formula I are able to form. Preferred are the pharmaceutically acceptable salts. Salts that are not pharmaceutically acceptable are also embraced in the scope of the present invention, since they refer to intermediates that may be useful in the preparation of compounds with pharmacological activity.

The salts can conveniently be obtained by treating the base form of the compounds of Formula I with such appropriate acids as inorganic acids such as hydrochloric or hydrobromic acid, sulfuric, nitric, phosphoric and the like acids; or organic acids such as, for example, acetic, propanoic, hydroxyacetic, lactic, pyruvic, oxalic (i.e. ethanedioic), malonic, succinic (i.e. butanedioic acid), maleic, fumaric, malic (i.e. hydroxybutanedioic acid), tartaric, citric, methanesulfonic, ethanesulfonic, benzenesulfonic, p-toluenesulfonic, cyclamic, salicylic, p-aminosalicylic, pamoic and the like acids.

The pharmaceutically acceptable salts can be obtained by treating the base form of the compounds of Formula I with such appropriate pharmaceutically acceptable acids like inorganic acids, for example, including hydrochloric, hydrobromic and the like; sulfuric acid; nitric acid; phosphoric acid and the like; or organic acids, for example, acetic, propanoic, hydroxyacetic, 2-hydroxypropanoic, 2-oxopropanoic, oxalic, malonic, succinic, maleic, fumaric, malic, tartaric, 2-hydroxy-1,2,3-propane-tricarboxylic, methanesulfonic, ethanesulfonic, benzenesulfonic, 4-methylbenzenesulfonic, cyclohexanesulfamic, 2-hydroxybenzoic, 4-amino-2-hydroxybenzoic and the like acids.

Conversely the salt form can be converted by treatment with alkali into the free base form.

The term pharmaceutical composition means for the purpose of present invention any composition which comprises as active compound, to whom is attributed, fully or in part, the therapeutical (pharmaceutical) effect, at least one of the compounds of the invention or combinations thereof and that may optionally further comprise at least one pharmaceutically acceptable non-active ingredient, as an excipient, carrier or so.

The term "preventing" refers to keep from happening, existing, or alternatively delaying the onset or recurrence of a disease, disorder, or condition to which such term applies, or of one or more symptoms associated with a disease, disorder, or condition. The term "prevention" refers to the act of preventing, as "preventing" is defined immediately above.

The term "treating", as used herein, refers to reversing, alleviating, or inhibiting the progress of the disorder or condition to which such term applies, or one or more symptoms of such disorders or condition. The term "treatment" refers to the act of treating, as "treating" is defined immediately above.

The term "subject" means animals, in particular mammals such as dogs, cats, cows, horses, sheep, geese, and humans. Particularly preferred subjects are mammals, including humans of both sexes.

An "effective amount" of the compounds of Formula I and pharmaceutically acceptable salts or prodrugs thereof, may be in the range from 0.01 mg to 50 g per day, from 0.02 mg to 40 g per day, from 0.05 mg to 30 g per day, from 0.1 mg to 20 g per day, from 0.2 mg to 10 g per day, from 0.5 mg to 5 g per day, from 1 mg to 3 g per day, from 2 mg to 2 g per day, from 5 mg to 1,5 g per day, from 10 mg to 1 g per day, from 10 mg to 500 mg per day.

Nerve cells include those cells from any region of the brain, spinal cord, optic nerve, retina, and peripheral ganglia. Neurons include those in embryonic, fetal, or adult neural tissue, including tissue from the hippocampus, cerebellum, spinal cord, cortex (e.g., motor or somatosensory cortex), striatum, basal forebrain (cholinergic neurons), ventral mesencephalon (cells of the substantia nigra), and the locus ceruleus (neuroadrenaline cells of the central nervous system).

In one embodiment of the present invention, the compounds of Formula I, its pharmaceutically acceptable salts and/or prodrugs thereof may be used for the prevention or treatment of one or more, preferably two or more, pathological or harmful conditions related to nerve cell death or damage selected from, but not being limited to, chemical substances such as oxidative stress conditions, toxic substances, infectious organisms, radiation, traumatic injury, hypoxia, ischemia, abnormal misfolded proteins, excitotoxins, free radicals, endoplasmic reticulum stressors, mitochondrial stressors including but not limited to inhibitors of the electron transport chain, Golgi apparatus antagonists, axonal damage or loss, demyelination, inflammation, pathological neuronal burst (seizures). Also preferably, the uses and methods of the present invention are directed to preventing or treating nerve cell death or damage, regardless of cause.

The terms "neuroprotection", "neuroprotective", or "neuroprotective effect" refer to the ability to prevent or reduce death or damage to nerve cells, including neurons and glia, or rescuing, resuscitating or reviving nerve cells, e.g., following in pathological or harmful conditions to the brain, central nervous system or peripheral nervous system. Thus, this neuroprotective effect comprises the conferred ability of neuronal cells to maintain or recover their neuronal functions. It stabilizes the cell membrane of a neuronal cell or helps in the normalization of neuronal cell functions. It prevents the loss of viability or functions of neuronal cells. It comprises the inhibition of progressive deterioration of neurons that leads to cell death. It refers too to any detectable protection of neurons from stress. Neuroprotection includes the regeneration of nerve cells, i.e. the re-growth of a population of nerve cells after disease or trauma.

Currently the majority of the neurological and psychiatric diseases lacks specific treatments aimed to stop or ameliorate the course of the disease, which are called "disease modifying drugs". This contrast with the symptomatic therapy which is common for such diseases but do not change the course of the disease. A neuroprotective drug is a Disease Modifying Drug (DMD) for the treatment of brain diseases.

As such, in one embodiment, the present invention relates to the use of the compounds of Formula I, its pharmaceutically acceptable salts and/or prodrugs thereof as active ingredient in the manufacture of a medicament for the regeneration of nerve cells. In other words, the present invention relates to the compounds of Formula I, its pharmaceutically acceptable salts and/or prodrugs thereof for use for the regeneration of nerve cells. Similarly, the present invention relates to a method of regenerating nerve cells comprising administering to a subject in need thereof an effective amount of the compounds of Formula I , its pharmaceutically acceptable salts and/or prodrugs thereof.

Neuroprotection may be determined directly by, for example, measuring the delay or prevention of neuronal death, such as, for example, by a reduction in the number of apoptotic neurons in cerebrocortical cultures following a stress. Neuroprotection may also be determined directly by, for example, measuring the severity or extent of damage to, or functional loss by, a tissue or organ of the nervous system following such a stress, such as, for example, by measuring a decrease in the size of brain infarcts after occlusion of the middle cerebral artery (MCAO) or reperfusion injury. Also, neuroprotection can be identified by magnetic resonance imaging (measuring brain volume, tractography, levels of N-acetyl-aspartate by spectroscopy). Alternatively, neuroprotection may be determined indirectly by detecting the activation of one or more biological mechanisms for protecting neurons, including, but not limited to, detecting activation of the Keap1/Nrf2 pathway or induction of one or more phase 2 enzymes, including but not limited to hemeoxygenase-1 (HO-1). Methods of detecting and measuring neuronal protection are provided in the Examples below, and other such methods are known in the art.

The various uses and methods employing the compounds of Formula I, its pharmaceutically salts and/or prodrugs thereof in the present invention comprise acute administration, i.e. occurring within several minutes to about several hours from injury, or chronic administration, suitable for chronic neurological or psychiatric diseases.

In one embodiment of the present invention, in the various uses and methods of neuroprotection or of prevention or treatment of nerve cell death or damage, the compounds of Formula I, its pharmaceutically acceptable salts and/or prodrugs thereof is administered to a subject with a neurological or psychiatric disease.

Neurological diseases are those disorders of the central and peripheral nervous system, including disorders of the brain, spinal cord, cranial nerves, peripheral nerves, nerve roots, autonomic nervous system, neuromuscular junction, and muscle.

Diseases of the central and peripheral nervous system, which may be subject of prevention and/or treatment according to present invention include, without being limited to, as knowledge in clinical manifestations advances, Absence of the Septum Pellucidum, Acid Lipase Disease , Acid Maltase Deficiency, Acquired Epileptiform Aphasia, Acute Disseminated Encephalomyelitis, Adie's Pupil, Adie's Syndrome, Adrenoleukodystrophy, Agenesis of the Corpus Callosum, Agnosia, Aicardi Syndrome, Aicardi-Goutieres Syndrome Disorder, AIDS - Neurological Complications, Alexander Disease, Alpers' Disease, Alternating Hemiplegia, Alzheimer's Disease, Amyotrophic Lateral Sclerosis, Anencephaly, Aneurysm, Angelman Syndrome, Angiomatosis, Anoxia, Antiphospholipid Syndrome, Aphasia, Apraxia, Arachnoid Cysts, Arachnoiditis, Arnold-Chiari Malformation, Arteriovenous Malformation, Asperger Syndrome, Ataxia, Ataxia Telangiectasia, Ataxias and Cerebellar or Spinocerebellar Degeneration, Atrial Fibrillation and Stroke, Attention Deficit-Hyperactivity Disorder (ADHD), Autism, Autonomic Dysfunction, Back Pain, Barth Syndrome, Batten Disease, Becker's Myotonia, Behcet's Disease, Bell's Palsy, Benign Essential Blepharospasm, Benign Focal Amyotrophy, Benign Intracranial Hypertension, Bernhardt-Roth Syndrome, Binswanger's Disease, Blepharospasm, Bloch-Sulzberger Syndrome, Brachial Plexus Birth Injuries, Brachial Plexus Injuries, Bradbury-Eggleston Syndrome, Brain and Spinal Tumors, Brain Aneurysm, Brain infarction, Brain ischemia, Brain Injury, Brown-Sequard Syndrome, Bulbospinal Muscular Atrophy, , CADASIL, Canavan Disease, Carpal Tunnel Syndrome, Causalgia, Cavernomas, Cavernous Angioma, Cavernous Malformation, Central Cervical Cord Syndrome, Central Cord Syndrome, Central Pain Syndrome, Central Pontine Myelinolysis, Cephalic Disorders, Ceramidase Deficiency, Cerebellar Degeneration, Cerebellar Hypoplasia, Cerebral Aneurysm, Cerebral Arteriosclerosis, Cerebral Atrophy, Cerebral Beriberi, Cerebral Cavernous Malformation, Cerebral Gigantism, Cerebral Hypoxia, Cerebral Palsy, Cerebro-Oculo-Facio-Skeletal Syndrome, Charcot-Marie-Tooth Disease, Chiari Malformation, Cholesterol Ester Storage **Disease, Chorea,** Choreoacanthocytosis, Chronic Inflammatory Demyelinating Polyneuropathy (CIDP), Chronic Orthostatic Intolerance, Chronic Pain, Cockayne Syndrome Type II, Coffin Lowry Syndrome, COFS, Colpocephaly, Coma, Complex Regional Pain Syndrome, Congenital Facial Diplegia, Congenital Myasthenia, Congenital Myopathy, Congenital Vascular Cavernous Malformations, Corticobasal Degeneration, Cranial Arteritis, Craniosynostosis, Creutzfeldt-Jakob Disease, Cumulative Trauma Disorders, Cushing's Syndrome, Cytomegalic Inclusion Body Disease, Cytomegalovirus Infection, Dancing Eyes-Dancing Feet Syndrome, Dandy-Walker Syndrome, Dawson Disease, De Morsier's Syndrome, Deep Brain Stimulation for Parkinson's Disease, Dejerine-Klumpke Palsy, Dementia, Dementia - Multi-Infarct, Dementia - Semantic, Dementia - Subcortical, Dementia With Lewy Bodies, Dentate Cerebellar Ataxia, Dentatorubral Atrophy, Dermatomyositis, Developmental Dyspraxia, Devic's Syndrome, Diabetic Neuropathy, Diffuse Sclerosis, Dravet Syndrome, Dysautonomia, Dysgraphia, Dyslexia, Dysphagia, Dyspraxia, Dyssynergia Cerebellaris Myoclonica, Dyssynergia Cerebellaris Progressiva, Dystonias, , Early Infantile Epileptic Encephalopathy, Empty Sella Syndrome, Encephalitis, Encephalitis Lethargica, Encephaloceles, Encephalopathy, Encephalopathy, familial infantile, with intracranial calcification and chronic cerebrospinal fluid lymphocytosis; Cree encephalitis; Pseudo-Torch syndrome; Pseudotoxoplasmosis syndrome, Encephalotrigeminal Angiomatosis, Epilepsy, Epileptic Hemiplegia, Erb-Duchenne and Dejerine-Klumpke Palsies, Erb's Palsy, Essential Tremor, Extrapontine Myelinolysis, Fabry Disease, Fahr's Syndrome, Fainting, Familial Dysautonomia, Familial Hemangioma, Familial Idiopathic Basal Ganglia Calcification, Familial Periodic Paralyses, Familial Spastic Paralysis, Farber's Disease, Febrile Seizures, Fibromuscular Dysplasia, Fisher Syndrome, Floppy Infant Syndrome, Foot Drop, Friedreich's Ataxia, Frontotemporal Dementia , Gangliosidoses, Gaucher's Disease, Gerstmann's Syndrome, Gerstmann-Straussler-Scheinker Disease, Giant Axonal Neuropathy, Giant Cell Arteritis, Giant Cell Inclusion Disease, Globoid Cell Leukodystrophy, Glossopharyngeal Neuralgia, Glycogen Storage Disease, Guillain-Barré Syndrome, Hallervorden-Spatz Disease, Head Injury, Headache, Hemicrania Continua, Hemifacial Spasm, Hemiplegia Alterans, Hereditary Neuropathies, Hereditary Spastic Paraplegia, Heredopathia Atactica Polyneuritiformis, Herpes Zoster, Herpes Zoster Oticus, Hirayama Syndrome, Holmes-Adie syndrome , Holoprosencephaly, HTLV-1 Associated Myelopathy, Hughes Syndrome, Huntington's Disease, Hydranencephaly, Hydrocephalus, Hydrocephalus - Normal Pressure, Hydromyelia, Hypercortisolism, Hypersomnia, Hypertonia, Hypotonia, Hypoxia, Immune-Mediated Encephalomyelitis, Inclusion Body Myositis, Incontinentia Pigmenti, Infantile Hypotonia, Infantile Neuroaxonal Dystrophy , Infantile Phytanic Acid Storage Disease, Infantile Refsum Disease, Infantile Spasms, Inflammatory Myopathies, Iniencephaly, Intestinal Lipodystrophy, Intracranial Cysts, Intracranial Hypertension, Isaac's Syndrome, Joubert Syndrome, Kearns-Sayre Syndrome, Kennedy's Disease, Kinsbourne syndrome, Kleine-Levin Syndrome, Klippel-Feil Syndrome, Klippel-Trenaunay Syndrome (KTS), Klüver-Bucy Syndrome, Korsakoffs Amnesic Syndrome, Krabbe Disease, Kugelberg-Welander Disease, Kuru, Lambert-Eaton Myasthenic Syndrome, Landau-Kleffner Syndrome, Lateral Femoral Cutaneous Nerve Entrapment, Lateral Medullary Syndrome, Learning Disabilities, Leigh's Disease, Lennox-Gastaut Syndrome, Lesch-Nyhan Syndrome, Leukodystrophy, Levine-Critchley Syndrome, Lewy Body Dementia, Lipid Storage Diseases, Lipoid Proteinosis, Lissencephaly, Locked-In Syndrome, Lou Gehrig's Disease, Lupus - Neurological Sequelae, Lyme Disease - Neurological Complications, Machado-Joseph Disease, Macrencephaly, Megalencephaly, Melkersson-Rosenthal Syndrome, Meningitis, Meningitis and Encephalitis, Menkes Disease, Meralgia Paresthetica, Metachromatic Leukodystrophy, Microcephaly, Migraine, Miller Fisher Syndrome, Mild Cognitive Impairment, Mini-Strokes, Mitochondrial Myopathies, Moebius Syndrome, Monomelic Amyotrophy, Motor Neuron Diseases, Moyamoya Disease, Mucolipidoses, Mucopolysaccharidoses, Multifocal Motor Neuropathy, Multi-Infarct Dementia, Multiple Sclerosis, Multiple System Atrophy, Multiple System Atrophy with Orthostatic Hypotension, Muscular Dystrophy, Myasthenia - Congenital, Myasthenia Gravis, Myelinoclastic Diffuse Sclerosis, Myoclonic Encephalopathy of Infants, Myoclonus, Myopathy, Myopathy - Congenital, Myopathy - Thyrotoxic, Myotonia, Myotonia Congenita, Narcolepsy, Neuroacanthocytosis, Neurodegeneration with Brain Iron Accumulation, Neurofibromatosis, Neuroleptic Malignant Syndrome, Neurological Complications of AIDS, Neurological Complications of Lyme Disease, Neurological Consequences of Cytomegalovirus Infection, Neurological Manifestations of Pompe Disease, Neurological Sequelae Of Lupus, Neuromyelitis Optica , Neuromyotonia, Neuronal Ceroid Lipofuscinosis, Neuronal Migration Disorders, Neuropathy - Hereditary, Neurosarcoidosis, Neurotoxicity, Nevus Cavernosus, Niemann-Pick Disease, Normal Pressure Hydrocephalus, Occipital Neuralgia, Ohtahara Syndrome, Olivopontocerebellar Atrophy, Opsoclonus Myoclonus, Orthostatic Hypotension, O'Sullivan-McLeod Syndrome, Overuse Syndrome, Pain - Chronic, Pantothenate Kinase-Associated Neurodegeneration, Paraneoplastic Syndromes, Paresthesia, Parkinson's Disease, Paroxysmal Choreoathetosis, Paroxysmal Hemicrania, Parry-Romberg, Pelizaeus-Merzbacher Disease, Pena Shokeir II Syndrome, Perineural Cysts, Periodic Paralyses, Peripheral Neuropathy, Periventricular Leukomalacia, Persistent Vegetative State, Pervasive Developmental Disorders, Phytanic Acid Storage Disease, Pick's Disease, Pinched Nerve, Piriformis Syndrome, Pituitary Tumors, Polymyositis, Pompe Disease, Porencephaly, Postherpetic Neuralgia, Postinfectious Encephalomyelitis, Post-Polio Syndrome, Postural Hypotension, Postural Orthostatic Tachycardia Syndrome, Postural Tachycardia Syndrome, Primary Dentatum Atrophy, Primary Lateral Sclerosis, Primary Progressive Aphasia, Prion Diseases, Progressive Hemifacial Atrophy, Progressive Locomotor Ataxia, Progressive Multifocal Leukoencephalopathy, Progressive Sclerosing Poliodystrophy, Progressive Supranuclear Palsy, Prosopagnosia, Pseudotumor Cerebri, Ramsay Hunt Syndrome I (formerly known as), Ramsay Hunt Syndrome II (formerly known as), Rasmussen's Encephalitis, Reflex Sympathetic Dystrophy Syndrome, Refsum Disease, Refsum Disease - Infantile, Repetitive Motion Disorders, Repetitive Stress Injuries, Restless Legs Syndrome, Retrovirus-Associated Myelopathy, Rett Syndrome, Reye's Syndrome, Rheumatic Encephalitis, Riley-Day Syndrome, Sacral Nerve Root Cysts, Saint Vitus Dance, Salivary Gland Disease, Sandhoff Disease, Schilder's Disease, Schizencephaly, Seitelberger Disease, Seizure Disorder, Semantic Dementia, Septo-Optic Dysplasia, Severe Myoclonic Epilepsy of Infancy (SMEI), Shaken Baby Syndrome, Shingles, Shy-Drager Syndrome, Sjögren's Syndrome, Sleep Apnea, Sleeping Sickness, Sotos Syndrome, Spasticity, Spina Bifida, Spinal Cord Infarction, Spinal Cord Injury, Spinal Cord Tumors, Spinal Muscular Atrophy, Spinocerebellar Atrophy, Spinocerebellar Degeneration, Steele-Richardson-Olszewski Syndrome, Stiff-Person Syndrome, Striatonigral Degeneration, Stroke, Sturge-Weber Syndrome, Subacute Sclerosing Panencephalitis, Subcortical Arteriosclerotic Encephalopathy, SUNCT Headache, Swallowing Disorders, Sydenham Chorea, Syncope, Syphilitic Spinal Sclerosis, Syringohydromyelia, Syringomyelia, Systemic Lupus Erythematosus, Tabes Dorsalis, Tardive Dyskinesia, Tarlov Cysts, Tay-Sachs Disease, Temporal Arteritis, Tethered Spinal Cord Syndrome, Thomsen's Myotonia, Thoracic Outlet Syndrome, Thyrotoxic Myopathy, Tic Douloureux, Todd's Paralysis, Tourette Syndrome, Transient Ischemic Attack, Transmissible Spongiform Encephalopathies, Transverse Myelitis, Traumatic Brain Injury, Tremor, Trigeminal Neuralgia, Tropical Spastic Paraparesis, Troyer Syndrome, Tuberous Sclerosis, Vascular Erectile Tumor, Vasculitis Syndromes of the Central and Peripheral Nervous Systems , Von Economo's Disease, Von Hippel-Lindau Disease (VHL), Von Recklinghausen's Disease, Wallenberg's Syndrome, Werdnig-Hoffman Disease, Wernicke-Korsakoff Syndrome, West Syndrome, Whiplash, Whipple's Disease, Williams Syndrome, Wilson's Disease, Wolman's Disease , X-Linked Spinal and Bulbar Muscular Atrophy, Zellweger Syndrome, optic neuritis, Chronic fatigue syndrome, fibromialgia, psychiatric diseases such as mood disorders, major depression, bipolar syndrome, psycosis, eschizophrenia, obsessive-compulsive-syndrome, etc., Toxic or drug abuse diseases such as alcoholism and drug abuse, Encephalopathy like hepatic encephalopathy.

Psychiatric disorders, which may be subject of prevention and/or treatment according to present invention include those listed by the Diagnostic and Statistical Manual of Mental Disorders, Fourth Edition (DSM-IV) published by the American Psychiatric Association, and covers all mental health disorders for both children and adults. In particular, psychiatric disorders include a disorder selected from Acute Stress Disorder; Adjustment Disorder Unspecified; Adjustment Disorder with Anxiety; Adjustment Disorder with Depressed Mood; Adjustment Disorder with Disturbance of Conduct; Adjustment Disorder with Mixed Anxiety and Depressed Mood; Adjustment Disorder with Mixed Disturbance of Emotions and Conduct; Agoraphobia without History of Panic Disorder; Anorexia Nervosa; Antisocial Personality Disorder; Anxiety Disorder Due to Medical Condition; Anxiety Disorder, NOS; Avoidant Personality Disorder; Bipolar Disorder NOS; Bipolar I Disorder, Most Recent Episode Depressed, In Full Remission; Bipolar I Disorder, Most Recent Episode Depressed, In Partial Remission; Bipolar I Disorder, Most Recent Episode Depressed, Mild; Bipolar I Disorder, Most Recent Episode Depressed, Moderate; Bipolar I Disorder, Most Recent Episode Depressed, Severe With Psychotic Features; Bipolar I Disorder, Most Recent Episode Depressed, Severe Without Psychotic Features; Bipolar I Disorder, Most Recent Episode Depressed, Unspecified; Bipolar I Disorder, Most Recent Episode Manic, In Full Remission; Bipolar I Disorder, Most Recent Episode Manic, In Partial Remission; Bipolar I Disorder, Most Recent Episode Manic, Mild; Bipolar I Disorder, Most Recent Episode Manic, Moderate; Bipolar I Disorder, Most Recent Episode Manic, Severe With Psychotic Features; Bipolar I Disorder, Most Recent Episode Manic, Severe Without Psychotic Features; Bipolar I Disorder, Most Recent Episode Manic, Unspecified; Bipolar I Disorder, Most Recent Episode Mixed, In Full Remission; Bipolar I Disorder, Most Recent Episode Mixed, In Partial Remission; Bipolar I Disorder, Most Recent Episode Mixed, Mild; Bipolar I Disorder, Most Recent Episode Mixed, Moderate; Bipolar I Disorder, Most Recent Episode Mixed, Severe With Psychotic Features; Bipolar I Disorder, Most Recent Episode Mixed, Severe Without Psychotic Features; Bipolar I Disorder, Most Recent Episode Mixed, Unspecified; Bipolar I Disorder, Most Recent Episode Unspecified; Bipolar I Disorder, Most Recent Episode Hypomanic; Bipolar I Disorder, Single Manic Episode, In Full Remission; Bipolar I Disorder, Single Manic Episode, In Partial Remission; Bipolar I Disorder, Single Manic Episode, Mild; Bipolar I Disorder, Single Manic Episode, Moderate; Bipolar I Disorder, Single Manic Episode, Severe With Psychotic Features; Bipolar I Disorder, Single Manic Episode, Severe Without Psychotic Features; Bipolar I Disorder, Single Manic Episode, Unspecified; Bipolar II Disorder; Body Dysmorphic Disorder; Borderline Personality Disorder; Breathing-Related Sleep Disorder; Brief Psychotic Disorder; Bulimia Nervosa; Circadian Rhythm Sleep Disorder; Conversion Disorder; Cyclothymic Disorder; Delusional Disorder; Dependent Personality Disorder; Depersonalization Disorder; Depressive Disorder NOS; Dissociative Amnesia; Dissociative Disorder NOS; Dissociative Fugue; Dissociative Identity Disorder; Dyspareunia; Dyssomnia NOS; Dyssomnia Related to (Another Disorder); Dysthymic Disorder; Eating Disorder NOS; Exhibitionism; Female Dyspareunia Due to Medical Condition; Female Hypoactive Sexual Desire Disorder Due to Medical Condition; Female Orgasmic Disorder; Female Sexual Arousal Disorder; Fetishism; Frotteurism; Gender Identity Disorder in Adolescents or Adults; Gender Identity Disorder in Children; Gender Identity Disorder NOS; Generalized Anxiety Disorder; Histrionic Personality Disorder; Hypoactive Sexual Desire Disorder; Hypochondriasis; Impulse -Control Disorder NOS; Insomnia Related to (Another Disorder); Intermittent Explosive Disorder; Kleptomania; Major Depressive Disorder, Recurrent, In Full Remission; Major Depressive Disorder, Recurrent, In Partial Remission; Major Depressive Disorder, Recurrent, Mild; Major Depressive Disorder, Recurrent, Moderate; Major Depressive Disorder, Recurrent, Severe With Psychotic Features; Major Depressive Disorder, Recurrent, Severe Without Psychotic Features; Major Depressive Disorder, Recurrent, Unspecified; Major Depressive Disorder, Single Episode, In Full Remission; Major Depressive Disorder, Single Episode, In Partial Remission; Major Depressive Disorder, Single Episode, Mild; Major Depressive Disorder, Single Episode, Moderate; Major Depressive Disorder, Single Episode, Severe With Psychotic Features; Major Depressive Disorder, Single Episode, Severe Without Psychotic Features; Major Depressive Disorder, Single Episode, Unspecified; Male Dyspareunia Due to Medical Condition; Male Erectile Disorder; Male Erectile Disorder Due to Medical Condition; Male Hypoactive Sexual Desire Disorder Due to Medical Condition; Male Orgasmic Disorder; Mood Disorder Due to Medical Condition; Narcissistic Personality Disorder; Narcolepsy; Nightmare Disorder; Obsessive Compulsive Disorder; Obsessive-Compulsive Personality Disorder; Other Female Sexual Dysfunction Due to Medical Condition; Other Male Sexual Dysfunction Due to Medical Condition; Pain Disorder Associated with both Psychological Factors and Medical Conditions; Pain Disorder Associated with Psychological Features; Panic Disorder with Agoraphobia; Panic Disorder without Agoraphobia; Paranoid Personality Disorder; Paraphilia, NOS; Parasomnia NOS; Pathological Gambling; Pedophilia; Personality Disorder NOS; Posttraumatic Stress Disorder; Premature Ejaculation; Primary Hypersomnia; Primary Insomnia; Psychotic Disorder Due to Medical Condition, with Delusions; Psychotic Disorder Due to Medical Condition, with Hallucinations; Psychotic Disorder, NOS; Pyromania; Schizoaffective Disorder; Schizoid Personality Disorder; Schizophrenia, Catatonic Type; Schizophrenia, Disorganized Type; Schizophrenia, Paranoid Type; Schizophrenia, Residual Type; Schizophrenia, Undifferentiated Type; Schizophreniform Disorder; Schizotypal Personality Disorder; Sexual Aversion Disorder; Sexual Disorder NOS; Sexual Dysfunction NOS; Sexual Masochism; Sexual Sadism; Shared Psychotic Disorder; Sleep Disorder Due to A Medical Condition, Hypersomnia Type; Sleep Disorder Due to A Medical Condition, Insomnia Type; Sleep Disorder Due to A Medical Condition, Mixed Type; Sleep Disorder Due to A Medical Condition, Parasomnia Type; Sleep Terror Disorder; Sleepwalking Disorder; Social Phobia; Somatization Disorder; Somatoform Disorder NOS; Specific Phobia; Transvestic Fetishism; Trichotillomania; Undifferentiated Somatoform Disorder; Vaginismus; and Voyeurism.

Preferably, as NGF agonists, the compounds of Formula I, its pharmaceutically acceptable salts and/or prodrugs thereof, can be used in the treatment of diseases wherein NGF has been proven effective in the state of the art, either in vivo or in vitro, due to their improving effects on cell differentiation and cell survival, throughout TrkA pathway. Therefore, the compounds covered in present invention can be used in the treatment of neurological diseases selected among: neurodegenerative disorders, such as amyotrophic lateral sclerosis (ALS), Parkinson's disease, Alzheimer's disease, Friedreich's ataxia, Huntington's disease, Dementia with Lewy bodies, spinal muscular atrophy; nerve inflammation, such as multiple sclerosis, neuromyelitis optica, major depressive disorder, schizophrenia, glaucoma; or peripheral neuropathies, such as diabetic or AIDS neuropathy. Moreover, the compounds of the invention can also be indicated for treatment of cancer, by modulating NGF cell differentiation activity and stopping cell proliferation. Among the cancer types in which NGF has been proven effective in the state of the art, either in vivo or in vitro, due to their improving effects on cell differentiation and cell survival, throughout either TrkA and/or p75 pathways, the following may be cited: glioblastoma, astrocytoma, meduloblastoma, neurinoma, neuroblastoma, meningioma, colon cancer, pancreatic cancer, breast cancer, prostate cancer, leukemia, acute lymphocytic leukemia, osteosarcoma, hepatocellular carcinoma, ovarian carcinoma, lung adenocarcinoma or esophagic carcinoma.

In one embodiment of the present invention, in the various uses and methods of neuroprotection or of prevention or treatment of nerve cell death or damage, the compounds of Formula I, its pharmaceutically acceptable salts and/or prodrugs thereof is administered to a healthy subject, preferably a healthy subject older than 18 years old, more preferably a healthy subject older than 45 years old, even more preferably a healthy subject older than 55, 60, 65, 70, 75, 80, 85, 90, 95, or 100 years old.

The term "healthy subject" is meant to comprise its plain meaning as well as those subjects that may suffer from one or more pathological conditions other than a neurological or psychiatric disease.

The neuroprotective properties of the compounds of Formula I, its pharmaceutically salts and/or prodrugs thereof have as a consequence the partial or full prevention or treatment of the various disorders in the nervous system functions caused by the neuronal cell death or damage. Therefore, the present invention further relates to the use of the compounds of Formula I, its pharmaceutically acceptable salts and/or prodrugs thereof as active ingredient in the manufacture of a medicament for the prevention or treatment of a neurological or psychiatric disease. In other words, the present invention also relates to the compounds of Formula I, its pharmaceutically acceptable salts and/or prodrugs thereof for use in the prevention or treatment of a neurological or psychiatric disease. Similarly, the present invention also relates to a method of prevention or treatment of a neurological or psychiatric disease comprising administering to a subject in need thereof an effective amount of the compounds of Formula I, its pharmaceutically acceptable salts and/or prodrugs thereof. The neurological or psychiatric disease may be any one from those listed above.

Preferably, the disease treated or prevented with the administration of the compounds of the invention is selected from: neurodegenerative disorders, such as amyotrophic lateral sclerosis (ALS), Parkinson's disease, Alzheimer's disease, Friedreich's ataxia, Huntington's disease, Dementia with Lewy bodies, spinal muscular atrophy; inflammation, such as multiple sclerosis, neuromyelitis optica, major depressive disorder, schizophrenia, glaucoma; or peripheral neuropathies, such as diabetic or AIDS neuropathy; and cancer, such as glioblastoma, astrocytoma, meduloblastoma, neurinoma, neuroblastoma, meningioma, colon cancer, pancreatic cancer, breast cancer, prostate cancer, leukemia, acute lymphocytic leukemia, osteosarcoma, hepatocellular carcinoma, ovarian carcinoma, lung adenocarcinoma esophagic carcinoma.

Another goal of present invention is the use of the compounds of Formula I, its pharmaceutically acceptable salts and/or prodrugs thereof, as neuroenhancing drugs or its use for manufacturing neuroenhancing drugs.

Neuroenhancing drugs include those that improve learning and memory, attention, mood, communicative skills and sexual performance. Examples of neuroenhancing drugs are those that target long-term synaptic potentiation (LTP) or long-term depression (LTD), modulation of calcium channels, or the cAMP response elementbinding (CREB) protein. cAMP is the acronym for cyclic adenosine monophosphate. Particular examples of neuroenhancing drugs are phosphodiesterase inhibitors like rolipram; donepezil; agonists of the NMDA glutamate receptor like D-cycloserine; ampakines; modafinil; methylphenidate.

The compounds of Formula I, its pharmaceutically acceptable salts and/or prodrugs thereof may be formulated into various pharmaceutical forms for administration purposes. As appropriate compositions there may be cited all compositions usually employed for systemically administering drugs, for example any solid (e.g. tablets, capsules, granules, etc.) or liquid composition (e.g. solutions, suspensions, emulsions, etc). To prepare the pharmaceutical compositions of the compounds of Formula I, an effective amount of the compounds of Formula I, optionally in salt form or a prodrug, as the active ingredient is combined in intimate admixture with a pharmaceutically acceptable carrier, which carrier may take a wide variety of forms depending on the form of preparation desired for administration. These pharmaceutical compositions are desirable in unitary dosage form suitable, particularly, for administration orally, rectally, percutaneously, intrathecal, intravenous or by parenteral injection. For example, in preparing the compositions in oral dosage form, any of the usual pharmaceutical media may be employed such as, for example, water, glycols, oils, alcohols and the like in the case of oral liquid preparations such as suspensions, syrups, elixirs, emulsions and solutions; or solid carriers such as starches, sugars, kaolin, lubricants, binders, disintegrating agents and the like in the case of powders, pills, capsules, and tablets. Because of their ease in administration, tablets and capsules represent the most advantageous oral dosage unit forms, in which case solid pharmaceutical carriers are obviously employed. For parenteral compositions, the carrier usually comprises sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. Also included are solid form preparations, which are intended to be converted, shortly before use, to liquid form preparations. In the compositions suitable for percutaneous administration, the carrier optionally comprises a penetration enhancing agent or a suitable wetting agent, or both, optionally combined with suitable additives of any nature in minor proportions, which additives do not introduce a significant deleterious effect on the skin. A review of the different pharmaceutical forms for drug administration and their preparation may be found in the book "Tratado de Farmacia Galénica", de C. Faulí i Trillo, 10th Edition, 1993, Luzán 5, S.A. de Ediciones.

It is especially advantageous to formulate the aforementioned pharmaceutical compositions in unit dosage form for ease of administration and uniformity of dosage. Unit dosage form as used herein refers to physically discrete units suitable as unitary dosages, each unit containing a predetermined quantity of active ingredient calculated to produce the desired therapeutic effect in association with the required pharmaceutical carrier. Examples of such unit dosage forms are tablets (including scored or coated tablets), capsules, pills, suppositories, powder packets, wafers, injectable solutions or suspensions and the like, and segregated multiples thereof.

The compositions in accordance with this invention, including unit dosage forms, may contain the active ingredient in an amount that is in the range of about 0,1 % to 70%, or about 0,5% to 50%, or about 1 % to 25%, or about 5% to 20%, the remainder comprising the carrier, wherein the foregoing percentages are w/w versus the total weight of the composition or dosage form.

The dose of the compounds of Formula I, its pharmaceutically acceptable salts and/or prodrugs thereof to be administered depends on the individual case and, as customary, is to be adapted to the conditions of the individual case for an optimum effect. Thus it depends, of course, on the frequency of administration and on the potency and duration of action of the compound employed in each case for therapy or prophylaxis, but also on the nature and severity of the disease and symptoms, and on the sex, age, weight co-medication and individual responsiveness of the subject to be treated and on whether the therapy is acute or prophylactic. Doses may be adapted in function of weight and for pediatric applications. Daily doses may be administered q.d. or in multiple quantities such as b.i.d., t.i.d. or q.i.d.

### Figure Legends

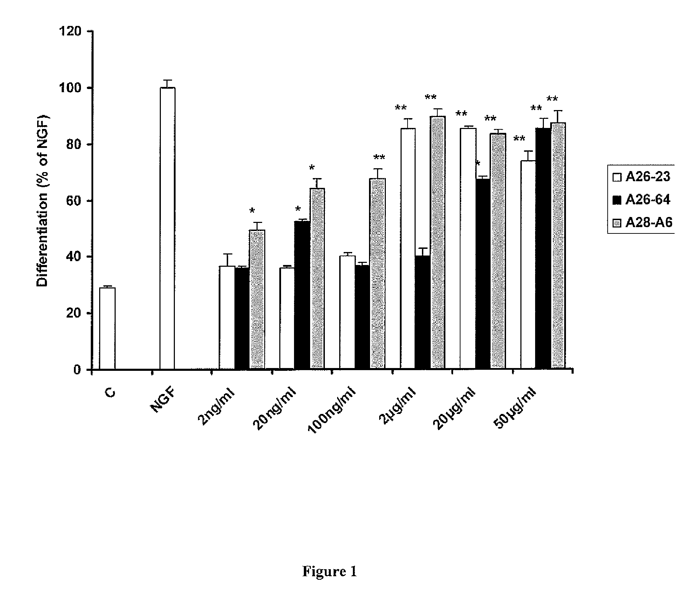

**Figure 1****.** Induction of PC12 cells differentiation by NGF-like molecules. Differentiation levels induced after 3 days of treatment with the 3-oxopiperazinium derivatives: A26-23, A26-64 and A28-A6. Data are expressed as percentage of the differentiation (cells with neurite processes at least 2 times the diameter of the cell body) induced by NGF. Data are the mean ± SEM of at least three experiments, each in duplicate. *p < 0.05, **p < 0.01 (ANOVA) with regard to the control.

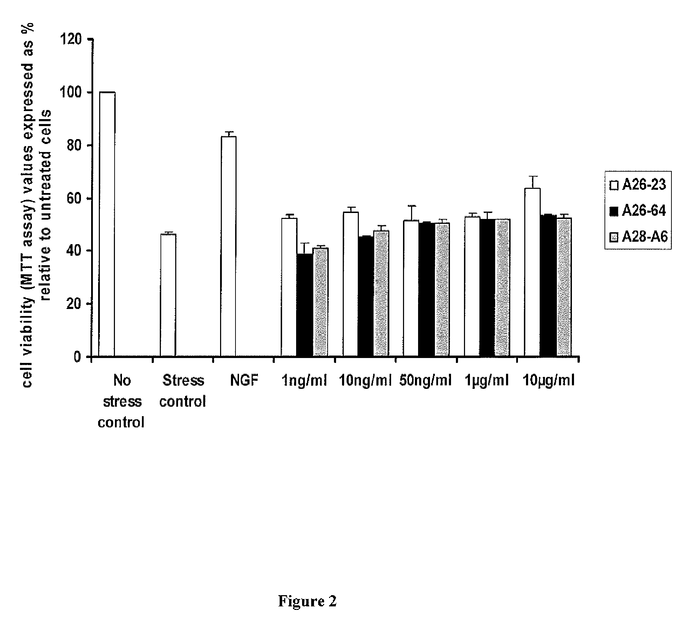

**Figure 2****.** Effects of NGF-like molecules in promotion of RN22 cell survival. RN22 schwannoma cells in serum free medium were treated with cupper sulphate (CuS04) (150 µM) to generate stress and cell death. After that NGF (100 ng/ml) or NGF-like small compounds derived from 3-oxopiperazinium: A26-23, A26-64 and A28-A6 were added at different concentrations. Cell viability was analyzed by MTT assay 24 h later. Depicted are the means ± S.E. of three experiments, each in duplicate. *p < 0.05, **p < 0.01 (ANOVA) respect to stress control.

### Detailed description of the invention

The invention uses a library (Masip et al., 2004) of tetraalkylammonium heterocyclic compounds, (100 individual molecules).

From the tested molecules the invention selected 3 compounds which showed a good NGF like activity "in vitro" by inducing differentiation of PC12 cells and therefore, having neuroregenerative properties.

The general Markush formula covering the compounds of the invention is the following: 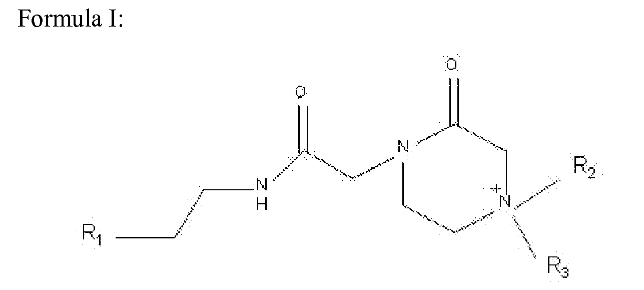 wherein 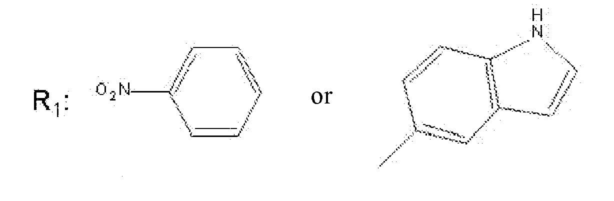 R₂ and R₃ are each one independently 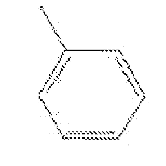 or CH₃
or form together with the 3- oxopiperazinium ring a fused azoniasapiro [4,5] decane heterocycle. The invention also covers pharmaceutically acceptable salts and/or prodrugs of the compounds of Formula I.

Preferred compounds according to present invention are those of Formula I in which, when 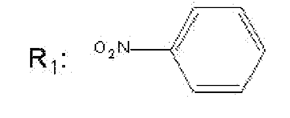 then R₂ and R₃ are each one independently CH₃
; or R₂ is CH₃ and R₃ is

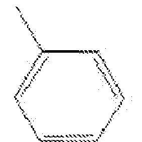

Also preferred compounds comprised in the invention are those of Formula I wherein when R₁ is 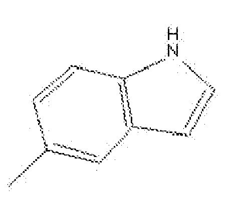 then R₂ and R₃ form together with the 3-oxopiperazinium ring a fused azoniasapiro [4,5] decane heterocycle. The invention also covers pharmaceutically acceptable salts and/or prodrugs of the above mentioned preferred compounds of Formula I.

As a way of example, preferred compounds according to present invention are selected among the following: 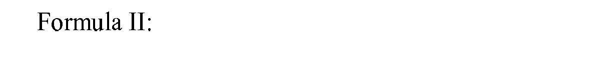 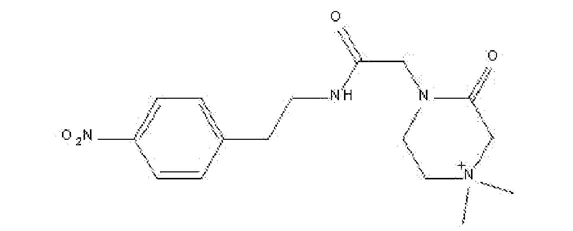 1,1- dimethyl-4-[2-(4-nitrophenethylamino) -2- oxoethyl] -3-oxopiperazinium chloride (A26-23) 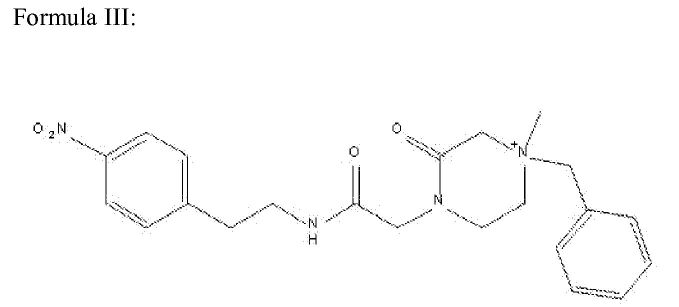 1-benzyl-1-methyl-4-[2-(4-nitrophenethylamino)-2-oxoethyl]-3-oxopiperazinium chloride (A26-64) 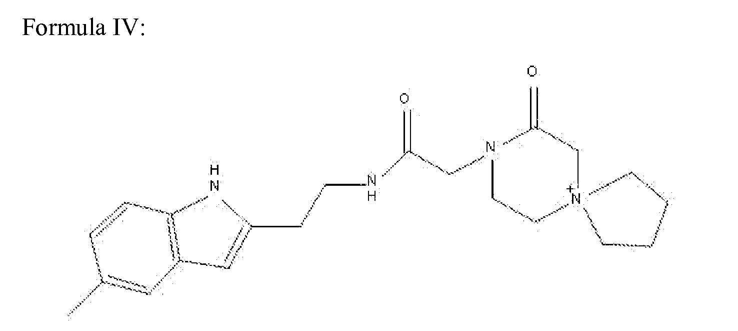 8-[2-(2-(5-methyl-1H-indol-2-yl)ethylamino)-2-oxoethyl]-7-oxo-8-aza-5-azoniaspiro[4.5]decane. (A28-A6);
and its pharmaceutically acceptable salts and/or prodrugs thereof.

The following examples are intended to illustrate the present invention and not to limit it thereto.

### Example 1: Design of NGF agonists by combinatorial chemistry

*General.* Solvents, amines and other reagents were purchased from commercial suppliers and used without further purification. Reaction carried out under microwave irradiation were conducted in a 100 mL round bottomed flask equipped with a Dimroth condenser. The flask was introduced in the monomode cavity of a CEM Model Discover apparatus. The NMR spectra were recorded on a Varian Inova 500 apparatus (¹H NMR, 500 MHz; ¹³C NMR, 125 MHz) and on a Unity 300 apparatus (¹H NMR, 300 MHz; ¹³C NMR, 75 MHz). When appropriate, the assignment of ¹H and ¹³C NMR peaks for compounds were confirmed by gDQCOSY and gHSQC experiments. The ocurrence of different conformers led to highly complex spectra; the absorptions given below are referred to the major conformer present in the sample. The RP-HPLC analyses were performed with a Hewlett Packard Series 1100 (UV detector 1315A) modular system using a reverse-phase Kromasil 100 C8 (25 x 0.46 cm, 5 µm) column, with CH₃CN-buffer ammonium formate (20 mM, pH=5.0) mixtures at 1 mL/min as mobile phase and monitoring at 220 nm. Semi-preparative RP-HPLC was performed with a Waters (Milford, MA, U.S.A.) system. High resolution mass spectra (HRMS-FAB) were carried at the Mass Spectrometry Service of the IQAC - Instituto de Quimica Avanzada de Cataluña - (Spain).

**Library of tetraalkylammonium derivatives.** This library of discrete compounds was designed and constructed as previously reported (Masip et al., 2004).

### Preparation of individual 3-oxopiperazinium derivatives for the in vivo assays.

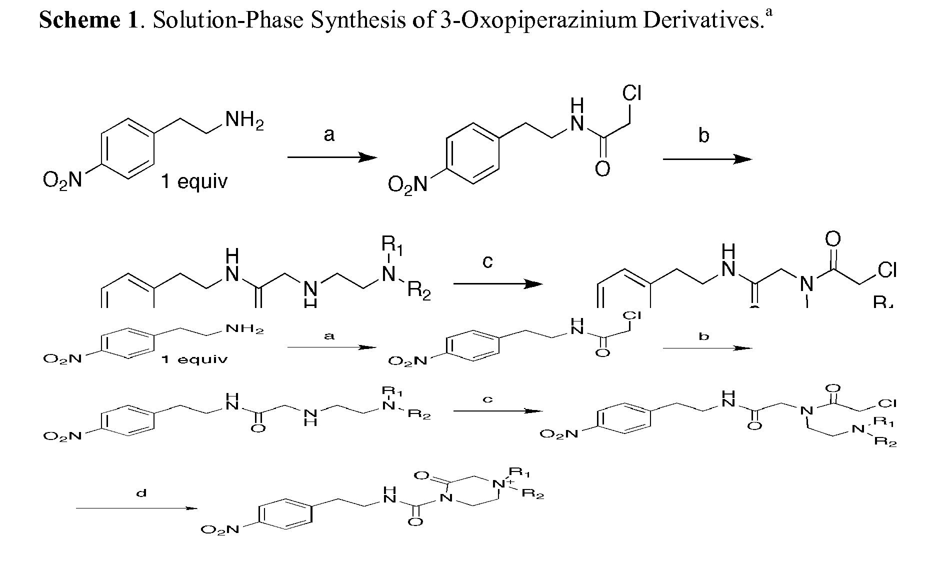

^{a}Reagents and conditions: (a) HOOCCH₂Cl, DIC, HOBt, DIPEA, DCM; (b) H₂NCH₂CH₂R¹R², DIPEA, dioxane; (c) ClCOCH₂Cl, DCM; (d) Al₂O₃, DCM-MeOH (2:1).

The individual tetraalkylammonium salts A26-23 and A26-64 for the in vivo assays were prepared similary to the corresponding library, but improving each step in order to achieve the highest yields and purities. In the case of A26-64, it was necessary to prepare N-benzyl-N-methylethane-1,2-diamine. This compound was obtained from the corresponding alcohol precursor by the Mitsunobu procedure, followed by hydrazinolysis. (Sen, S. et al, 1995).

**Synthesis of *N*-benzyl-*N*-methylethane-1,2-diamine.** To a solution of N-benzyl-N-methylethanolamine (7.31 mL, 40.5 mmol), phtalimide (6.62 g, 44.5 mmol), and triphenyl phosphine (14 g, 52.65 mmol) in anhydrous THF (240 mL), cooled at 0 °C, diisopropyl diazacarboxylate (12.73 mL, 60.75 mmol) was added dropwise in the absence of ligth. The mixture was stirred for 15 h at 20°C and then the solvent was removed under reduced pressure. The expected phtalimide derivative was isolated in 80% yield (70% purity) after repeated treatment with 1:1 AcOEt:Et₂O mixtures to remove Ph₃P and Ph₃PO by preciptation. The phtalimide derivative (16.8 g, 39 mmol) was redissolved in absolute ethanol (200 mL) treated with hydrazine hydrate (9.7 mL, 195 mmol), and the mixture was stirred for 6 h at 80 °C. The crude reaction mixture was acidified with 1.5 N HCl and the resulting suspension was filtered. The solvents were removed under reduced pressure, the residue was redissolved in CH₂Cl₂ and the amine was extracted with 1.5 N HCl. This acid solution was taken to pH with NaOH and extracted with CH₂Cl₂. The combined organic fractions were washed (brine), dried (MgSO₄) and concentrated in vacuo. The N -benzyl-*N*-methylethane-1,2-diamine was purified by vacuum distillation as a colorless oil (4.3 g, 88% yield, 95% purity). ¹H NMR (300 MHz, CDCl3) ∂ 7.3-7.2 (ca, 5H, H_{Ar}), 3.50 (s, 2H, NCH₂Ph), 2.79 (t, ³*J*_{HH}=6 Hz, 2H, CH₂NH₂), 2.45 (t, ³*J*_{HH}=6 Hz, 2H, CH₂N), 2.20 (s, 3H, CH₃N), 1.62 (NH₂); ¹³C NMR (75 MHz, CDCl₃) ∂ 128.9 (CH_{Ar}, 2,6-CH), 128.2 (CH_{Ar}, 3,5-CH), 126.9 (CH_{Ar}, 4-CH), 62.6 (NCH₂Ph), 60.0 (CH₂N), 42.1 (CH₃N), 39.5 (CH₂NH₂); HRMS calcd for C₁₀H₁₆N₂ 165.1392 (M + H)+, found 165.1390.

**Synthesis of *N*-(4-nitrophenethyl)-2-chloroacetamide.** A suspension of 2-(4-nitrophenyl)ethylamine hydrochloride (3.5 g, 17 mmol) in 30 mL anhydrous CH₂Cl₂ was stirred with *N,N*-diisopropylethylamine (2.94 mL, 17 mmol). Chloroacetic acid (1.60 g mmol, 17.0 mmol) in 10 mL anhidride DCM was added dropwise to the stirred solution containing the free amine maintained at 0 °C and under argon atmosphere, followed by the addition of (*N*,*N*'-diisopropylcarbodiimide, 2.93 mL, 18.7 mmol) and 1-hydroxybenzotriazole hydrate (2.3 g, 18.7 mmol). Then, the mixture was stirred for 2 h at 20 °C. The crude reaction mixture was cooled at 0 °C, filtered and washed with 2 N HCl, brine and dried. Elimination of solvent yielded the expected *N*-substituted chloroacetamide (7.4 g, 90% yield, 95% purity). ¹H NMR (300 MHz, CDCl₃) ∂ 8.18 (d, ³*J*_{HH}=8.5 Hz, 2H, 3,5-H C₆H₄NO₂), 7.37 (d, ³*J*_{HH}=8.5 Hz, 2H, 2,6-H C₆H₄NO₂), 6.64 (s, 1H, NH), 4.04 (s, 2H, COCH₂Cl), 3.61 (q, ³*J*_{HH}=7 Hz, 2H, CH₂NH), 2.97 (t, ³*J*_{HH}=7 Hz, 2H, CH₂CH₂NH); ¹³C NMR (75 MHz, CDCl₃) ∂ 165.3 (NHCO), 146.9 (1,4-C C₆H₄NO₂), 129.6 (2,6-CH C₆H₄NO₂), 123.9 (3,5-CH C₆H₄NO₂), 43.5 (CH₂Cl), 40.8 (CH₂NH), 35.4 (CH₂CH₂NH).

### Preparation of 1,1-dimethyl-4-[2-(4-nitrophenethylamino)-2-oxoethyl]-3-oxopiperazinium chloride (A26-23).

*Coupling of N,N'-dimethylethylenediamine.* A mixture of the *N*-substituted chloroacetamide (2.97 g, 11.6 mmol), N,N'-dimethylethylenediamine (7.1 mL, 62 mmol) and *N,N*-diisopropylethylamine (4.3 mL, 24.7 mmol) in dioxane (25 mL) was allowed to react under microwave irradiation with the CEM reactor equipped with a Dimroth condenser (10 min, 80 °C, 150 W). Then the solvent was removed under reduced pressure to give the corresponding glycinamide (5.05 g crude, 98% yield, 71% purity by HPLC). ¹H NMR (400 MHz, (CD₃)₂O) ∂ 8.17 (d, ³*J*_{HH}=8.5 Hz, 2H, 3,5-H C₆H₄NO₂), 7.57 (d, ³*J*_{HH}=8.5 Hz, 2H, 2,6-H C₆H₄NO₂), 3.43 (q, ³*J*_{HH}=7 Hz, 2H, CH₂ CH₂NH), 3.15 (s, 2H, COCH₂NH), 3.03 (t, ³*J*_{HH}=7 Hz, 2H, CH₂CH₂NH), 2.76 (t, ³*J*_{HH}=7 Hz, 2H, NHCH₂CH₂N), 2.48 (s, 6H, 2 x CH₃N), 2.25 (t, ³*J*_{HH}=7 Hz, 2H, NHCH₂CH₂N); ¹³C NMR (75 MHz, CDCl₃) ∂ 165.3 (NHCO), 146.9 (1,4-C C₆H₄NO₂), 129.6 (2,6-CH C₆H₄NO₂), 123.8 (3,5-CH C₆H₄NO₂), 58.8 (CH₂CH₂N), 52.6 (COCH₂NH), 47.4 (CH₂CH₂N), 45.5 (2 x CH₃N), 39.7 (CH₂ CH₂NH), 35.8 (CH₂CH₂NH). Underlined atoms are the ones responsible of RMN signals detected herein.

*Acylation of the glycinamide and final cyclization.* Chloroacetyl chloride (1.41 mL, 17.75 mmol) was added to a stirred suspension of the glycinamide intermediate (5.0 g, 12.07 mmol) in anhydrous CH₂Cl₂ (20 mL) at 0 °C, and the mixture was stirred for 1 h, filtered and evaporated to dryness (7.4 g crude, 57% purity). The residue was redissolved in a 2:1 mixture of DCM/MeOH (50 mL), and the solution was treated with basic aluminum oxide (20 % w/w) for 15 h at 20 °C. The crude reaction mixture was filtered, and the solvent was removed to give the expected product as solid. The compound was washed by using subsequent mixtures of solvents to yield 1.6 g of the cyclic tetraalkylammonium compound (36% overall yield from starting amine, 95% purity). ¹H NMR (500 MHz, D₂O) ∂ 8.17 (d, ³*J*_{HH}=8.5 Hz, 2H, 3,5-H C₆H₄NO₂), 7.46 (d, ³*J*_{HH}=8.5 Hz, 2H, 2,6-H C₆H₄NO₂), 4.22 (s, 2H, COCH₂N⁺), 4.11 (s, 2H, COCH₂N), 3.84 (t, ³*J*_{HH}=6 Hz, 2H, NCH₂CH₂N⁺) , 3.72 (t, ³*J*_{HH}=6 Hz, 2H, NCH₂ CH₂N⁺), 3.55 (t, ³*J*_{HH}=7 Hz, 2H, CH₂ CH₂NH), 3.31 (s, 6H, 2 x CH₃N⁺), 2.95 (t, ³*J*_{HH}=7 Hz, 2H, CH₂CH₂NH); ¹³C NMR (100 MHz, D₂O) ∂ 168.9 (NHCO), 162.4 (NCO), 147.8 (1-C C₆H₄NO₂), 146.5 (4-C C₆H₄NO₂), 130.1 (2,6-CH C₆H₄NO₂), 123.8 (3,5-CH C₆H₄NO₂), 57.3 (COCH₂N⁺), 52.5 (2 x CH₃N⁺), 49.0 (CH₂CH₂N⁺), 42.9 (COCH₂N), 40.2 (CH₂CH₂N⁺), 35.8 (C_{Ar}CH₂CH₂), 34.8 (C_{Ar}CH₂CH₂); HRMS calcd for C₁₆H₂₃N₄O₄⁺ 335.1714 (M)+, found 335.1714.

### Preparation of 1-benzyl-1-methyl-4-[2-(4-nitrophenethylamino)-2-oxoethyl]-3-oxopiperazinium chloride (A26-64).

*Coupling of N-benzyl-¹-methylethane-1,2-diamine.* To a solution of the *N-*substituted chloroacetamide (1,01 g, 3.95 mmol) in anhydrous dioxane (20 mL), it was added *N*-benzyl-*N*-methylethane-1,2-diamine (0.64 mL, 4.2 mmol), anhydrous CsCO₃ (14 g, 43 mmol) and activated molecular sieves (Type A4). The mixture was allowed to react under microwave irradiation for 2 h by subsequent cycles of 10 min at 90 °C (150 W). The crude reaction mixture was filtered and the solid was washed with dioxane. Final product was isolated after elimination of solvent under vacuum (3.52 g, 77% yield, 60% purity). ¹H NMR (500 MHz, (CDCl₃) ∂ 8.16 (d, ³*J*_{HH} = 8.5 Hz, 2H, 3,5-H C₆H₄NO₂), 7.56 (t, ³*J*_{HH} = 7.5 Hz, 1H, 4-H C₆H₅), 7.34 (d, ³*J*_{HH} = 8.5 Hz, 2H, 2,6-H C₆H₄NO₂), 7.32-7.27 (4H, 2,3,5,6-H C₆H₅), 3.71 (s, 2H, NCH₂Ph), 3.55 (q, ³*J*_{HH} = 7 Hz, 2H, CH₂ CH₂NH), 3.18 (s, 2H, COCH₂NH), 2.93 (t, ³*J*_{HH} = 7 Hz, 2H, CH₂CH₂NH), 2.58 (t, ³*J*_{HH} = 5.5 Hz, 2H, NHCH₂CH₂N), 2.41 (t, ³*J*_{HH} = 5.5 Hz, 2H, NHCH₂CH₂N), 2.18 (s, 3H, CH₃N).

*Acylation of the glycinamide and final cyclization.* Acylation of this glycinamide was carried out as described for A26-23 to give 3.82 g of the crude product (88% yield, 58% purity). This crude was redissolved in 30 mL of a 2:1 DCM:MeOH mixture and treated with basic aluminum oxide (20 % w/w). The cyclization was performed under microwave activation for 20 min (30 °C, 150 W). The crude reaction mixture was filtered and concentrated under vacuum to give the expected product as as a solid. Purification was accomplished by extractions and reprecipitation in different solvents. Finally, 1.57 g, of the cyclic tetraalkylammonium compound were isolated (80% overall yield from starting amine, 95% purity). ¹H NMR (500 MHz, D₂O) ∂ 8.16 (d, ³*J*_{HH}=8.5 Hz, 2H, 3,5-H C₆H₄NO₂), 7.62 (tt, ³*J*_{HH}=7.5 Hz, |⁴*J*_{HH}|=2.5 Hz, 1H, 4-H C₆H₅), 7.57 (tt, ³*J*_{HH}=7.5 Hz, 2H, 3,5-H C₆H₅), 7.54 (tt, ³*J*_{HH}=7.5 Hz, 2H, 2,6-H C₆H₅), 7.45 (d, ³*J*_{HH}=8.5 Hz, 2H, 2,6-H C₆H₄NO₂), 4.73 (d, |²*J*_{HH}|=13 Hz, 1H, NCHₐ ,_{b}Ph), 4.65 (d, |²*J*_{HH}|=13 Hz, 1H, NCH_{a ,b}Ph), 4.26 (d, |²*J*_{HH}|=16.5 Hz, 1H, COCH_{a ,b}N⁺), 4.06 (d, |²*J*_{HH}|=16.5 Hz, 1H, COCH_{a,b}N⁺), 4.10 (d, |²*J*_{HH}|=3 Hz, 2H, COCH₂N), 3.82-3.7 (4H, NCH₂CH₂N⁺ + NCH₂CH₂N⁺), 3.54 (t, ³*J*_{HH}=6.5 Hz, 2H, CH₂ CH₂NH), 3.18 (s, 3H, CH₃N⁺), 2.94 (t, ³*J*_{HH}=6.5 Hz, 2H, CH₂CH₂NH); ¹³C NMR (100 MHz, D₂O) ∂ 168.8 (NHCO), 162.5 (NCO), 147.8 (1-C C₆H₄NO₂), 146.4 (4-C C₆H₄NO₂), 133.1 (2,6-CH C₆H₅), 131.4 (1-CH C₆H₅), 130.1 (2,6-CH C₆H₄NO₂), 129.5 (3,5-CH C₆H₅), 125.6 (4-C C₆H₅), 123.7 (3,5-CH C₆H₄NO₂), 69.0 (COCH₂N⁺), 59.6 (CH₂ C₆H₅), 54.8 (COCH₂N), 48.9 (CH₃N⁺), 47.9 (CH₂CH₂N⁺), 42.6 (CH₂CH₂N⁺), 40.1 (C_{Ar}CH₂CH₂), 34.8 (C_{Ar}CH₂CH₂); HRMS calcd for C₂₂H₂₇N₄O₄⁺ 411.2027 (M)+, found 411.2028.

### Preparation of 3-oxopiperazinium derivative A28-6 for the in vitro assays.

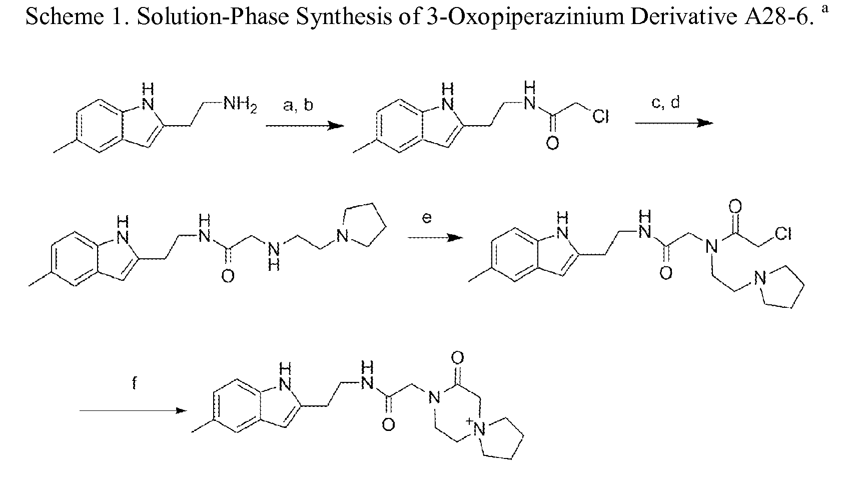

^{a} Reagents and conditions: (a) ClCOCH₂Cl, K₂CO₃, CH₂Cl₂, 0 °C, 30 min; (b) Wang aldehyde resin (3.5 equivalents/equivalents amine), dioxane, microwave irradiation, 40 min; (c) 2-(pyrrolidin-1-yl)ethanamine (3 equivalents), K₂CO₃ (3 equivalents), dioxane, 90 °C, 3h; (d) Wang aldehyde resin (3 equivalents/equivalents amine), dioxane, microwave irradiation, 20 min; (e) ClCOCH₂Cl (1 equivalents), K₂CO₃ (3 equivalents), CH₂Cl₂, 0 °C, 30 min; (f) Al₂O₃, CH2C12-MeOH (2:1).

**Synthesis of 8-[2-(2-(5-methyl-1*H*-indol-2-yl)ethylamino)-2-oxoethyl]-7-oxo-8-aza-5-azoniaspiro[4.5]decane. (A28-A6).** The individual tetraalkylammonium salt A28-A6 was obtained from the preparation of the library of tetraalkylammonium derivatives. The synthetic procedure involved a 6-step sequence carried out in solution, along with the use of solid-phase linked scavengers and microwave activation for the rapid removal of the excess of amine reagents.

*Synthesis of N-(5-methyltryptamine)-2-chloroacetamide.* Chloroacetyl chloride (16 µL, 0.2 mmol) was added dropwise to a stirred suspension of 5-methyltryptamine (52.3 mg, 0.3 mmol, 1.5 equivalents) and K₂CO₃ (55.3 mg, 0.4 mmol) in anhydrous CH₂Cl₂ maintained at 0 °C. The mixture was stirred at this temperature for 30 min, filtered and evaporated to render a residue which was redissolved in dioxane (0.5 mL) and treated with the 4-benzyloxybenzaldehyde polystyrene resin (Wang aldehyde HL) (0.35 mmol, 3.5 equivalents relative to the amine, 2.8 mmol/g). The reaction mixture was placed in a domestic microwave and irradiated (350 W) for 40 min at 4-min intervals. The crude reaction mixture was filtered, and the resin was washed with dioxane (2 mL) and CH₂Cl₂ (3 x 2 mL). The filtrate was evaporated to afford the corresponding N-substituted chloroacetamide.

*Coupling of the 2-(pyrrolidin-1-yl)ethanamine (A6).* The residue obtained was dissolved in dioxane (2 mL) and treated with the 2-(pyrrolidin-1-yl)ethanamine (76 µL, 0.6 mmol) and K₂CO₃ (55.3 mg, 0.4 mmol). The mixture was stirred at 90 °C for 3 h, filtered, and concentrated to 1 mL. This solution was treated with the Wang aldehyde HL resin (1.2 mmol, 3 equivalents relative to the amine, 2.8 mmol/g) for 20 min in the microwave oven (4-min intervals). The resin was filtered and washed with dioxane (2 mL) and CH₂Cl₂ (3 x 2 mL). The filtrate was evaporated to obtain the glycinamide.

*Acylation of the glycinamide.* Chloroacetyl chloride (16 µL, 0.2 mmol) was added to a stirred suspension of the glycinamide and K₂CO₃ (55.3 mg, 0.4 mmol) in CH₂Cl₂ (2.5 mL) at 0 °C, and the mixture was stirred for 30 min, filtered and evaporated to dryness.

*Cyclization.* The former residue was redissolved in a 2:1 mixture of CH₂Cl₂/MeOH (2 mL), and the solution was treated with basic aluminum oxide for 1 h at 20 °C. The crude reaction mixture was filtered, and the solvent was removed to give the corresponding cyclic tetraalkylammonium compound (57% overall yield from starting amine, 65% purity determined by HPLC-UV). The compound was identified by HPLC-MS.

### Example 2: PC12 cell differentiation A26-23, A26-24 and A28-A6 peptidomimetics induce differentiation.

PC 12 cell differentiation was measured by plating cells onto collagen-coated 24-wells plates. NGF (100 ng/ml) or the small chemicals at different concentrations were added and the percentage of cells with neurite processes greater than two cell bodies in length were counted after relevant treatment. For each experiment at least 300 cells were randomly measured (Burstein and Greene, 1978).

*Cell culture.* PC12 cells were maintained at 37°C in DMEM supplemented with 2.5% FBS, 15% of Horse serum (HS) and penicillin/streptomycin in a humidified 5% CO₂ incubator. The cells were grown on 60-and 100-mm tissue culture dishes (Becton Dickinson).

PC 12 cells were cultured for 3 days in the presence of the peptoids (from 2 ng/ml to 50 µg/ml) under reduced serum conditions (0.5% FBS and 1% HS). The peptoids A26-23, A26-24 and A28-A6 (Figure 1) were found to induce the differentiation of PC12 cells at different concentrations to an extent substantially comparable with that induce by NGF (Foehr et al., 2000) and showing a dose-response activity.

### Example 3: Survival assays A26-23, A26-24 and A28-A6 mimetic peptoids do not promote cell survival via p75 receptor.

Compounds identified from the initial library screening were also tested to assess their capacity to promote cell survival. To delineate p75 signalling independent of TrkA, we used a rat schwannoma cell line (RN22) expressing p75, but not TrkA, (Gentry et al., 2000).

*Cell culture.* The rat schawnnoma cell line RN22 was cultured in 5% CO₂ at 37°C in Dulbecco's modified Eagle's medium (DMEM) with 10% fetal bovine serum (FBS) and penicillin/streptomycin.

RN22 cells were plated at 20,000 cells/well on a 24-well plate in DMEM alone and after allowing the cells to adhere for 3 days, cupper sulphate (CuSO4) (150 µM) was added with or without NGF (100 ng/ml) or the peptidomimetic molecules A26-23, A26-24 and A28-A6 at different concentrations (1 ng/ml - 10 µg/ml) to generate stress and cell death. After 24 h cell viability was studied by determining the amount of yellow MTT (Sigma) that was reduced to insoluble purple formazan. After removing the medium, the water-insoluble formazan was solubilised with DMSO (Sigma), and the dissolved material was measured spectrophotometrically at a wavelength of 570 nm, subtracting the background at 650 nm (Frade, 2005).

After 24 h, cell viability was tested and peptidomimetic compounds A26-23, A26-24 and A28-A6 were found not to promote survival of RN22 cells at different concentrations (Figure 2), hence indicating that the effects of these peptidomimetics are not exerted via p75 receptor.

### References

Aloe L, Calzá L (2004) Progress in brain research. Vol 146, NGF and related molecules in health and diseases. Amsterdam: Elsevier.
Anand P. Neurotrophic factors and their receptors in human sensory neuropathies. Prog Brain Res. 2004;146:477-92.
Apfel S (2002) Nerve growth factor for the treatment of diabetic neuropathy: what went wrong, what went right, and what does the future hold? Int Rev Neurobiol 50:393-413.
Barker P (1998) p75NTR: A study in contrasts. Cell Death Differ 5:346-356.
Bhakar A, Howell J, Paul C, Salehi A, Becker E, Said F, Bonni A, Barker P (2003) Apoptosis induced by p75NTR overexpression requires Jun kinase-dependent phosphorylation of Bad. J Neurosci 23:11373-11381.
Burstein D, Greene L (1978) Evidence for RNA synthesis-dependent and - independent pathways in stimulation of neurite outgrowth by nerve growth factor. Proc Natl Acad Sci U S A 75:6059-6063.
Chao M (2003) Neurotrophins and their receptors: a convergence point for many signalling pathways. Nat Rev Neurosci 4:299-309.
Faden AI, Stoica B. Neuroprotection: challenges and opportunities. Arch Neurol. 2007 Jun;64(6):794-800.
Foehr E, Lin X, O'Mahony A, Geleziunas R, Bradshaw R, Greene W (2000) NF-kappa B signaling promotes both cell survival and neurite process formation in nerve growth factor-stimulated PC 12 cells. J Neurosci 20:7556-7563.
Frade J (2005) Nuclear translocation of the p75 neurotrophin receptor cytoplasmic domain in response to neurotrophin binding. J Neurosci 25:1407-1411.
Gentry J, Casaccia-Bonnefil P, Carter B (2000) Nerve growth factor activation of nuclear factor kappaB through its p75 receptor is an anti-apoptotic signal in RN22 schwannoma cells. J Biol Chem 275:7558-7565.
Ghosh S, May M, Kopp E (1998) NF-kappa B and Rel proteins: evolutionarily conserved mediators of immune responses. Annu Rev Immunol 16:225-260.
Greene L, Tischler A (1976) Establishment of a noradrenergic clonal line of rat adrenal pheochromocytoma cells which respond to nerve growth factor. Proc Natl Acad Sci U S A 73:2424-2428.
Hellweg R, Ziegenhorn A, Heuser I, Deuschle M. Serum concentrations of nerve growth factor and brain-derived neurotrophic factor in depressed patients before and after antidepressant treatment. Pharmacopsychiatry. 2008 Mar;41 (2):66-71.
Huang E, Reichardt L (2003) Trk receptors: roles in neuronal signal transduction. Annu Rev Biochem 72:609-642. Epub 2003 Mar 2027.
Kaplan D, Miller F (2000) Neurotrophin signal transduction in the nervous system. Curr Opin Neurobiol 10:381-391.
Levi-Montalcini R (1987) The nerve growth factor 35 years later. Science 237:1154-1162.
Lewin G, Barde Y (1996) Physiology of the neurotrophins. Annu Rev Neurosci 19:289-317.
Longo F, Manthorpe M, Xie Y, Varon S (1997) Synthetic NGF peptide derivatives prevent neuronal death via a p75 receptor-dependent mechanism. J Neurosci Res 48:1-17.
Longo FM, Yang T, Knowles JK, Xie Y, Moore LA, Massa SM. Small molecule neurotrophin receptor ligands: novel strategies for targeting Alzheimer's disease mechanisms. Curr Alzheimer Res. 2007 Dec;4(5):503-6.
Maliartchouk S, Debeir T, Beglova N, Cuello A, Gehring K, Saragovi H (2000a) Genuine monovalent ligands of TrkA nerve growth factor receptors reveal a novel pharmacological mechanism of action. J Biol Chem 275:9946-9956.
Maliartchouk S, Feng Y, Ivanisevic L, Debeir T, Cuello A, Burgess K, Saragovi H (2000b) A designed peptidomimetic agonistic ligand of TrkA nerve growth factor receptors. Mol Pharmacol 57:385-391.
Martinez-Forero I, Garcia-Munoz R, Martinez-Pasamar S, Inoges S, Lopez-Diaz de Cerio A, Palacios R, Sepulcre J, Moreno B, Gonzalez Z, Fernandez-Diez B, Melero I, Bendandi M, Villoslada P (2008) IL-10 suppressor activity and ex vivo Tr1 cell function are impaired in multiple sclerosis. Eur J Immunol 38:576-586.
Masip, I.; Ferrándiz-Huertas, C.; Garcia-Martinez, C.; Ferragut, J.; Ferrer-Montiel, A.; Messeguer, A. Synthesis of a Lybrary of 3-Oxopiperazinium and Perhydro-3-oxo-1,4-diazepinium Derivatives and Identification of Bioactive Compounds. J. Comb. Chem. 2004, 6, 135-141.
Miller S, Simon R, NG S, Zuckermann R, Kerr J, Moos W (1994) Bioorg Med Chem Letter 4:2657-2662.
Moreno B, Hevia H, Santamaria M, Sepulcre J, Munoz J, Garcia-Trevijano E, Berasain C, Corrales F, Avila M, Villoslada P (2006) Methylthioadenosine reverses brain autoimmune disease. Ann Neurol 60:323-334.
Palacios R, Comas D, Elorza J, Villoslada P (2008) Genomic regulation of CTLA4 and multiple sclerosis. J Neuroimmunol 203:108-115.
Peleshok J, Saragovi H (2006) Functional mimetics of neurotrophins and their receptors. Biochem Soc Trans 34:612-617.
Poduslo J, Curran G (1996) Permeability at the blood-brain and blood-nerve barriers of the neurotrophic factors: NGF, CNTF, NT-3, BDNF. Brain Res Mol Brain Res 36:280-286.
Price RD, Milne SA, Sharkey J, Matsuoka N. Advances in small molecules promoting neurotrophic function. Pharmacol Ther. 2007 Aug;115(2):292-306.
Rabizadeh S, Ye X, Wang J, Bredesen D (1999) Neurotrophin dependence mediated by p75NTR: contrast between rescue by BDNF and NGF. Cell Death Differ 6:1222-1227.
Saragovi H, Zaccaro M (2002) Small molecule peptidomimetic ligands of neurotrophin receptors, identifying binding sites, activation sites and regulatory sites. Curr Pharm Des 8:2201-2216.
Schulte-Herbrüggen O, Braun A, Rochlitzer S, Jockers-Scherübl MC, Hellweg R. Neurotrophic factors--a tool for therapeutic strategies in neurological, neuropsychiatric and neuroimmunological diseases? Curr Med Chem. 2007;14(22):2318-29.
Sen, S., Roach, S. A convenient Two-step procedure for the Synthesis of Substituted Allylic amines from Allylic Alcohols. Synthesis, 1995, 756-758.)
Shi Z, Birman E, Saragovi HU. Neurotrophic rationale in glaucoma: a TrkA agonist, but not NGF or a p75 antagonist, protects retinal ganglion cells invivo. Dev Neurobiol. 2007 Jun;67(7):884-94.
Shoval G, Weizman A. The possible role of neurotrophins in the pathogenesis and therapy of schizophrenia. Eur Neuropsychopharmacol. 2005 May;15(3):319-29.
Vajda FJ. Neuroprotection and neurodegenerative disease. J Clin Neurosci. 2002 Jan;9(1):4-8.
Villoslada P, Genain C (2004) Role of nerve growth factor and other trophic factors in brain inflammation. Prog Brain Res 146:403-414.
Villoslada P, Abel K, Heald N, Goertsches R, Hauser S, Genain C (2001) Frequency, heterogeneity and encephalitogenicity of T cells specific for myelin oligodendrocyte glycoprotein in naive outbred primates. Eur J Immunol 31:2942-2950.
Villoslada P, Hauser S, Bartke I, Unger J, Heald N, Rosenberg D, Cheung S, Mobley W, Fisher S, Genain C (2000) Human nerve growth factor protects common marmosets against autoimmune encephalomyelitis by switching the balance of T helper cell type 1 and 2 cytokines within the central nervous system. J Exp Med 191:1799-1806.
Williams B, Eriksdotter-Jonhagen M, Granholm A (2006) Nerve growth factor in treatment and pathogenesis of Alzheimer's disease. Prog Neurobiol 80:114-128. Epub 2006 Nov 2002.
Youdim MB, Buccafusco JJ. Multi-functional drugs for various CNS targets in the treatment of neurodegenerative disorders. Trends Pharmacol Sci. 2005 Jan;26(1):27-35.
Yoon S, Casaccia-Bonnefil P, Carter B, Chao M (1998) Competitive signaling between TrkA and p75 nerve growth factor receptors determines cell survival. J Neurosci 18:3273-3281.
Zaccaro M, Ivanisevic L, Perez P, Meakin S, Saragovi H (2001) p75 Co-receptors regulate ligand-dependent and ligand-independent Trk receptor activation, in part by altering Trk docking subdomains. J Biol Chem 276:31023-31029.

## Claims

1. A compound of formula I: 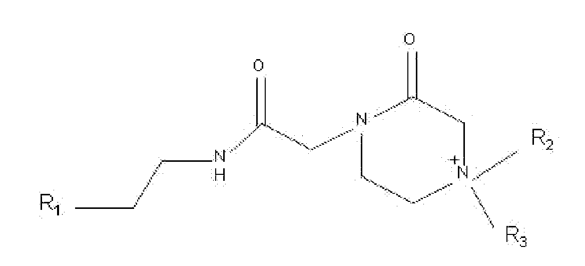 wherein: 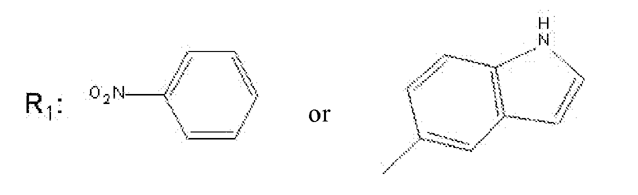 R₂ and R₃ are each one independently 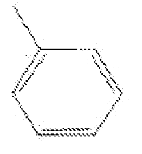 or CH₃
or form together with the 3-oxopiperazinium ring a fused azoniasapiro [4,5] decane heterocycle;
and pharmaceutically acceptable salts and/or prodrugs thereof.

2. A compound of Formula I, according to claim 1, wherein when 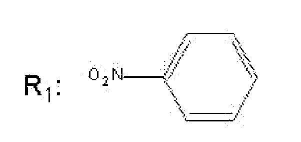 then R₂ and R₃ are each one independently CH₃;
or R₂ is CH₃ and R₃ is 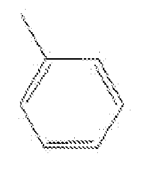 ; and pharmaceutically acceptable salts and/or prodrugs thereof.

3. A compound of Formula I, according to claim 1, wherein when R₁ is 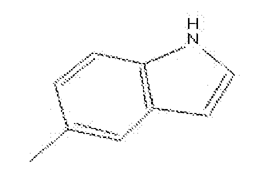 then R₂ and R₃ form together with the 3-oxopiperazinium ring a fused azoniasapiro [4,5] decane heterocycle;
and pharmaceutically acceptable salts and/or prodrugs thereof.

4. A compound according to any of the claims 1-3, selected among 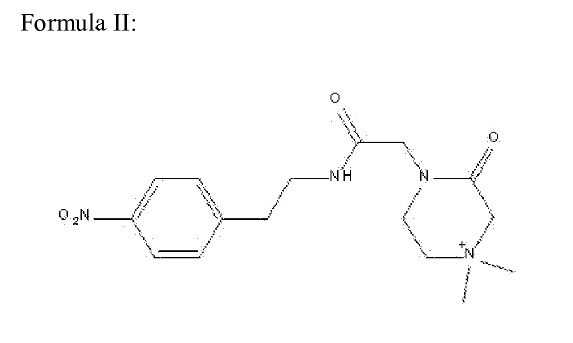 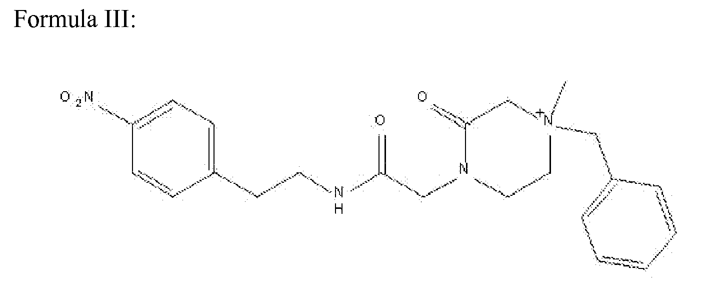 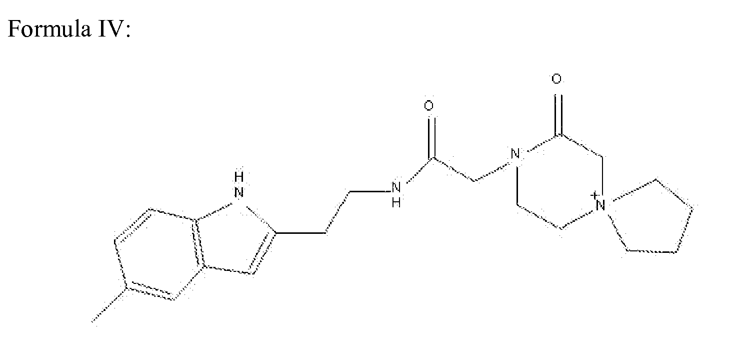 and pharmaceutically acceptable salts and/or prodrugs thereof.

5. The compounds of claims 1 to 4 for use as medicaments.

6. The compounds according to claim 5 for use as medicaments useful in the prevention or treatment of nerve cell death or damage.

7. The compounds according to claim 5 wherein the medicament is a neuroprotective drug.

8. The compounds according to claim 5 wherein the medicament is useful in the regeneration of nerve cells.

9. The compounds according to claim 5 wherein the medicament is a neuroenhancing drug.

10. The compounds according to claim 5 wherein the medicament is useful in the prevention or treatment of a neurological or psychiatric disease.

11. The compounds according to claim 5 wherein the medicament is useful in the prevention or treatment of a disease selected from: neurological diseases, preferentially neurodegenerative disorders, such as amyotrophic lateral sclerosis (ALS), Parkinson's disease, Alzheimer's disease, Friedreich's ataxia, Huntington's disease, Dementia with Lewy bodies, spinal muscular atrophy; nerve inflammation, such as multiple sclerosis, neuromyelitis optica, major depressive disorder, schizophrenia, glaucoma; or peripheral neuropathies, such as diabetic or AIDS neuropathy; and cancer, such as glioblastoma, astrocytoma, meduloblastoma, neurinoma, neuroblastoma, meningioma, colon cancer, pancreatic cancer, breast cancer, prostate cancer, leukemia, acute lymphocytic leukemia , osteosarcoma, hepatocellular carcinoma, ovarian carcinoma, lung adenocarcinoma , esophagic carcinoma.

12. Pharmaceutical compositions comprising a therapeutically effective amount of at least one of the compounds of claims 1 to 4 or combinations thereof and further comprising, optionally, at least one pharmaceutically acceptable non-active ingredient, preferably at least one excipient and/or carrier.

13. Use of the compounds of claims 1 to 4 as active ingredients in the manufacture of a medicament for the prevention or treatment of nerve cell death or damage.

14. Use of the compounds of claims 1 to 4 as active ingredients in the manufacture of a neuroprotective medicament.

15. Use of the compounds of claims 1 to 4 as active ingredients in the manufacture of a medicament for the regeneration of nerve cells.

16. Use of the compounds of claims 1 to 4 as active ingredients in the manufacture of a medicament for the prevention or treatment of a neurological or psychiatric disease

17. Use of the compounds of claims 1 to 4 as active ingredients in the manufacture of a medicament for the prevention or treatment of a disease selected from: neurological diseases, preferentially neurodegenerative disorders, such as amyotrophic lateral sclerosis (ALS), Parkinson's disease, Alzheimer's disease, Friedreich's ataxia, Huntington's disease, Dementia with Lewy bodies, spinal muscular atrophy; nerve inflammation, such as multiple sclerosis, neuromyelitis optica, major depressive disorder, schizophrenia, glaucoma; or peripheral neuropathies, such as diabetic or AIDS neuropathy; and cancer, such as glioblastoma, astrocytoma, meduloblastoma, neurinoma, neuroblastoma, meningioma, colon cancer, pancreatic cancer, breast cancer, prostate cancer, leukemia, acute lymphocytic leukemia , osteosarcoma, hepatocellular carcinoma, ovarian carcinoma, lung adenocarcinoma , esophagic carcinoma.

18. A method of prevention or treatment of nerve cell death or damage comprising administering to a subject in need thereof an effective amount of the compounds of claims 1 to 4; or of the pharmaceutical compositions of claim 12.

19. A method of neuroprotection comprising administering to a subject in need thereof an effective amount of the compounds of claims 1 to 4; or of the pharmaceutical compositions of claim 12.

20. A method of regenerating nerve cells comprising administering to a subject in need thereof an effective amount of the compounds of claims 1 to 4; or of the pharmaceutical compositions of claim 12.

21. A method of prevention or treatment of a disease comprising administering to a subj ect in need thereof an effective amount of the compounds of claims 1 to 4; or of the pharmaceutical compositions of claim 12, wherein the disease is selected from: neurological diseases, preferentially neurodegenerative disorders, such as amyotrophic lateral sclerosis (ALS), Parkinson's disease, Alzheimer's disease, Friedreich's ataxia, Huntington's disease, Dementia with Lewy bodies, spinal muscular atrophy; nerve inflammation, such as multiple sclerosis, neuromyelitis optica, major depressive disorder, schizophrenia, glaucoma; or peripheral neuropathies, such as diabetic or AIDS neuropathy; and cancer, such as glioblastoma, astrocytoma, meduloblastoma, neurinoma, neuroblastoma, meningioma, colon cancer, pancreatic cancer, breast cancer, prostate cancer, leukemia, acute lymphocytic leukemia, osteosarcoma, hepatocellular carcinoma, ovarian carcinoma, lung adenocarcinoma, esophagic carcinoma.
